# EUROPÄISCHE PATENTSCHRIFT

(11) **EP 2 630 947 B1**
(45) Veröffentlichungstag und Bekanntmachung des Hinweises auf die Patenterteilung: **25.07.2018**
(21) Anmeldenummer: 12198397.7
(22) Anmeldetag: 20.12.2012
(51) Int. Cl.: A61K 8/37, A61Q 13/00, A61Q 17/04, A61K 8/06

(54) **Duftstabile, wasserfeste kosmetische Zubereitung**
Stable perfume, waterproof cosmetic preparation
Préparation cosmétique imperméable et au parfum stable

(30) Priorität: 04.01.2012 DE 102012200071
(43) Veröffentlichungstag der Anmeldung: 28.08.2013
(73) Patentinhaber: Beiersdorf AG, 20253 Hamburg (DE)
(72) Erfinder: Zanforlin Trede, Lucia, 22529 Hamburg (DE); Blohm, Alexandra, 20257 Hamburg (DE); Möllgaard, Svenja Lena, 22337 Hamburg (DE); Pasternak, Anja, 22085 Hamburg (DE)

(56) Entgegenhaltungen:
- EP-A2- 0 427 411
- WO-A1-2004/045564
- DE-A1- 19 751 221
- FR-A1- 2 916 347

## Beschreibung

Die vorliegende Erfindung betrifft eine kosmetische Zubereitung enthaltend Esterwachs, Salicylate und Parfümstoffe.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Um kosmetische Zubereitungen, beispielsweise Sonnenschutzmittel, möglichst kostengünstig herstellen zu können werden Rohstoffe mit begrenztem Reinheitsgrad eingesetzt, die in Spuren häufig unangenehm riechende Nebenbestandteile enthalten.
Darüber hinaus haben eine Reihe von kosmetischen Inhaltsstoffen an sich einen unangenehmen Eigengeruch. Es besteht daher in der Regel die Notwendigkeit, die Zubereitungen ausreichend zu parfümieren. Nachteilig am Stande der Technik ist jedoch der Umstand, dass die Parfümierung nicht über die gesamte Lebensdauer des Produktes bzw. der Wz/prü-02883

Anwendung stabil bleibt. Insbesondere bei Sonnenschutzmitteln kommt darüber hinaus das Problem auf, dass sich Bestandteile der Zubereitungen bei der Anwendung in der Sonnenwärme und unter UV-Strahlung in übelriechende Fragmente zersetzen oder dass durch zeitlich ungleichmäßiges Verdampfen von Parfümbestandteilen aus der applizierten Zubereitung der Geruch der Zubereitung sich im Verlauf der Anwendung ändert. Dies kann beispielsweise bei den Parfümstoffen Linalool, Benzyl Benzoate, Hydroxyisohexyl 3-Cyclohexene Carboxaldehyd, Hexyl Cinnamal, Benzyl Salicylate, Citronellol, Coumarin, Limonene, Butylphenyl Methylpropional, Eugenol, Geraniol, Alpha-Isomethyl lonone eine Rolle spielen.

Es war daher die Aufgabe der vorliegenden Erfindung, die Nachteile des Standes der Technik zu beseitigen und die Geruchsstabilität/Duftstabilität kosmetischer Zubereitungen, insbesondere von Sonnenschutzmitteln zu verbessern.

Aufgrund ihrer gegenüber W/O-Emulsionen angenehmeren Sensorik und der Tatsache, dass sich mit ihnen generell höhere Lichtschutzfaktoren erzielen lassen, werden als Grundlage für Sonnenschutzmittel und Tagespflegeprodukte bevorzugt Öl-in-Wasser-Emulsionen (O/W-Emulsionen) eingesetzt. O/W-Emulsionen haben jedoch aufgrund der wässrigen äußeren Phase den Nachteil, nicht besonders wasserfest zu sein. Da aber Sonnenschutzmittel häufig am Strand oder Schwimmbad zum Einsatz kommen, also bei Aktivitäten, bei denen die Anwender mehr oder weniger intensiv mit Wasser in Kontakt kommen, besteht ein großer Bedarf an "wasserfesten" Sonnenschutzmitteln. Nach herkömmlichen Stand der Technik, wird versucht, die Wasserfestigkeit von Sonnenschutzmitteln, insbesondere auf O/W-Emulsionsbasis, dadurch zu erhöhen, dass man den Zubereitungen sogenannte Filmbildner zusetzt, die bei der Applikation auf der Haut, die UV-Filtersubstanzen fixieren sollen. Nachteilig an diesen Zubereitungen ist jedoch der Umstand, dass diese Filmbildner dazu führen, dass die Zubereitungen ein klebrig-schmieriges Hautgefühl verursachen, also sensorisch unattraktiv sind. Darüber hinaus führen diese Filmbildner dazu, dass die Sandanhaftung (oder Anhaftung anderer "Schmutzpartikel") auf der Haut erhöht wird.

Es war daher die Aufgabe der vorliegenden Erfindung, die Wasserfestigkeit von Sonnenschutzmitteln (und Tagespflegeprodukten) auf O/W-Emulsionsbasis auf der Haut zu verbessern, ohne dass die Zubereitungen ein klebrig-schmieriges Hautgefühl erzeugen und die Neigung zur Anhaftung von Sand oder anderen Partikeln reduziert ist.

Überraschend gelöst werden diese Aufgaben durch eine kosmetische Öl-in-Wasser Emulsion (O/W Emulsion) enthaltend
a) C18-C38 Alkylhydroxystearoylstearat,
b) ein oder mehrere UV-Lichtschutzfilter aus der Gruppe der Salicylate Ethylhexylsalicylat und Homomenthylsalicylat (INCI:Homosalate),
c) einen oder mehrere Parfümstoffe,
wobei die Emulsion eine Viskosität von 1000 bis 3000 mPas aufweist,
dadurch gekennzeichnet, dass als Emulgator Kaliumcetylphosphat und Glycerylstearat eingesetzt wird.

Die erfindungsgemäße Viskosität wird dabei mit Hilfe des folgenden Messverfahrens bestimmt: Zur Messung der Viskosität wird das Rotationsrheometer Rheomat R123 der Firma proRheo mit dem VT02- Messsystem verwendet. Die Proben werden vor der Messung für mindestens 24 Stunden in Ruhe bei 25°C gelagert.

Zwar kennt der Stand der Technik die EP 1534217, EP 0987001, EP 0987002, EP 0987006, EP 0987008, EP 1964540, EP 1477159, DE 19842730, DE 19842766 und DE 19751221, doch konnten diese Schriften nicht den Weg zur vorliegenden Erfindung weisen. Ferner kennt der Fachmann die EP 0427411, WO 2004/045564 und FR 2916347, die ebenfalls nicht den Weg zur vorliegenden Erfindung weisen konnten.

Es ist erfindungsgemäß, wenn die Zubereitung als Esterwachs C18-C38 Alkylhydroxystearoylstearat enthält. Dieses Esterwachs wird in der Kosmetik mit der INCI C18-38 Alkyl Hydroxystearoyl Stearate bezeichnet und ist beispielsweise bei der Fima Koster Keunen unter der Bezeichnung Kesterwachs K 80 P erworben werden.

Es ist erfindungsgemäß bevorzugt, wenn die erfindungsgemäße Zubereitung C18-C38 Alkylhydroxystearoylstearat in einer Gesamtkonzentration von 0,2 bis 2,5 Gewichts-%, bezogen auf das Gesamtgewicht der Zubereitung, enthält.

Es ist erfindungsgemäß besonders bevorzugt, wenn die erfindungsgemäße Zubereitung C18-C38 Alkylhydroxystearoylstearat in einer Gesamtkonzentration von 0,5 bis 1,5 Gewichts-%, bezogen auf das Gesamtgewicht der Zubereitung, enthält.

Es ist erfindungsgemäß, wenn als UV-Lichtschutzfilter aus der Gruppe der Salicylate Ethylhexylsalicylat (Salicylsäure-2-ethylhexylester) und/oder Homomenthylsalicylat (3,3,5-Trimethylcyclohexylsalicylat, lNCI:Homosalate) eingesetzt werden.

Erfindungsgemäß bevorzugte Ausführungsformen der vorliegenden Erfindung sind dadurch gekennzeichnet, dass die Zubereitung Ethylhexylsalicylat und Homomenthylsalicylat (INCI: Homosalate) in einer Gesamtkonzentration von 3 bis 15 Gewichts-%, bezogen auf das Gesamtgewicht der Zubereitung, enthält, wobei diese Gesamtkonzentration die Einzelkonzentration darstellt für den Fall, dass die Zubereitung nur eines der beiden Salicylate enthält.

Erfindungsgemäß besonders bevorzugte Ausführungsformen der vorliegenden Erfindung sind dadurch gekennzeichnet, dass die Zubereitung Ethylhexylsalicylat und Homomenthylsalicylat (INCI: Homosalate) in einer Gesamtkonzentration von 7 bis 15 Gewichts-%, bezogen auf das Gesamtgewicht der Zubereitung, enthält, wobei diese Gesamtkonzentration die Einzelkonzentration darstellt für den Fall, dass die Zubereitung nur eines der beiden Salicylate enthält.

Es ist erfindungsgemäß, wenn als Emulgator Kaliumcetylphosphat (INCI Potassium Cetyl Phosphate) und Glycerylstearat (INCI Glyceryl Stearate SE oder Glyceryl Stearate) eingesetzt wird.

Es ist erfindungsgemäß bevorzugt, wenn die Zubereitung Kaliumcetylphosphat und Glycerylstearat in einer Gesamtkonzentration von 0,15 bis 3,5 Gewichts-%, bezogen auf das Gesamtgewicht der Zusammensetzung, enthält, besonders bevorzugt in einer Gesamtkonzentration von 0,25 bis 2 Gewichts-%, bezogen auf das Gesamtgewicht der Zusammensetzung, enthält. Es ist vorteilhaft im Sinne der vorliegenden Erfindung, wenn die Zubereitung Ethanol enthält.

Dabei ist es erfindungsgemäß bevorzugt, wenn die Zubereitung Ethanol in einer Konzentration von 2 bis 10 Gewichts-%, bezogen auf das Gesamtgewicht der Zubereitung, enthält.

Es ist erfindungsgemäß besonders bevorzugt, wenn die Zubereitung Ethanol in einer Konzentration von 4 bis 8 Gewichts-%, bezogen auf das Gesamtgewicht der Zubereitung, enthält.

Erfindungsgemäß vorteilhafte Ausführungsformen der vorliegenden Erfindung sind dadurch gekennzeichnet, dass der oder die erfindungsgemäßen Parfümstoffe gewählt werden aus der Gruppe Linalool, Benzyl Benzoate, Hydroxyisohexyl 3-Cyclohexene Carboxaldehyd, Hexyl Cinnamal, Benzyl Salicylate, Citronellol, Coumarin, Limonene, Butylphenyl Methylpropional, Eugenol, Geraniol, Alpha-Isomethyl Ionone.

Dabei ist es erfindungsgemäß bevorzugt, wenn die erfindungsgemäßen Parfümstoffe ausgewählt werden aus der Gruppe der Verbindungen Coumarin, Limonen, Eugenol und Geraniol.

Es ist erfindungsgemäß besonders bevorzugt, wenn die Zubereitung Coumarin in einer Konzentration von 0,0002 bis 0,008 Gewichts-%, bezogen auf das Gesamtgewicht der Zubereitung, enthält.

Es ist erfindungsgemäß besonders bevorzugt, wenn die Zubereitung Limonen in einer Konzentration von 0,002 bis 0,08 Gewichts-%, bezogen auf das Gesamtgewicht der Zubereitung, enthält.

Es ist erfindungsgemäß besonders bevorzugt, wenn die Zubereitung Eugenol in einer Konzentration von 0,002 bis 0,04 Gewichts-%, bezogen auf das Gesamtgewicht der Zubereitung, enthält.

Es ist erfindungsgemäß besonders bevorzugt, wenn die Zubereitung Geraniol in einer Konzentration von 0,0002 bis 0,002 Gewichts-%, bezogen auf das Gesamtgewicht der Zubereitung, enthält.

Es ist erfindungsgemäß von Vorteil, wenn die erfindungsgemäße Zubereitung weitere UV-Lichtschutzfilter und/oder weitere Parfümstoffe enthält.

So sind erfindungsgemäß bevorzugte Ausführungsformen der vorliegenden Erfindung dadurch gekennzeichnet, dass die Zubereitung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 2-(4'-Diethylamino-2'-hydoxybenzoyl)-benzoesäurehexylester; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; 2-Ethylhexyl-2-hydroxybenzoat;; Dimethicodiethylbenzalmalonat; 3-(4-(2,2-bis Ethoxycarbonylvinyl)-phe-noxy)propenyl)-methoxysiloxan / Dimethylsiloxan - Copolymer; ) 2-Ethylhexyl-2-cyano-3,3-di-phenylacrylat (Octocrylen); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoesäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'-Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), Titandioxid, Zinkoxid, Merocyanine, Piperazinderivate, enthält.

Die erfindungsgemäß vorteilhaften Merocyanine werden dabei gewählt aus der Gruppe der Verbindungen

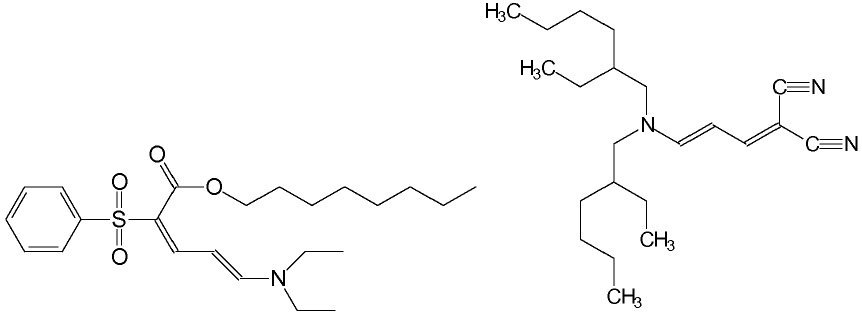

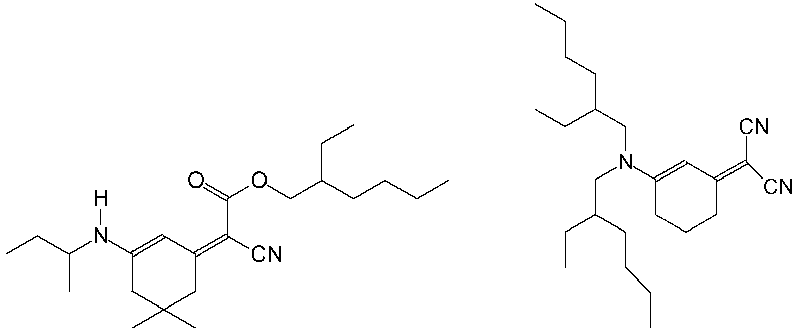

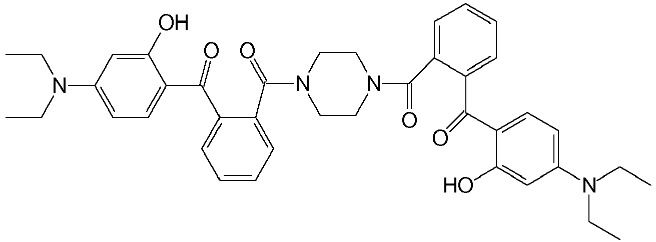

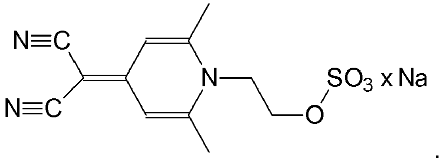

Als erfindungsgemäß vorteilhaftes Piperazinderivat kann dabei die folgende Verbindung eingesetzt werden:

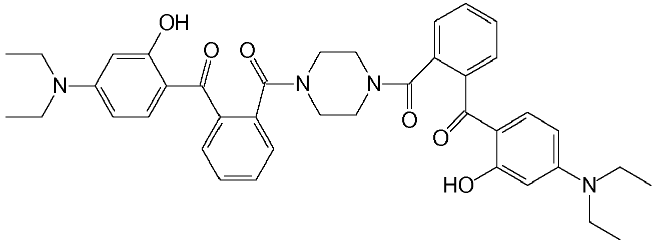

Es ist erfindungsgemäß vorteilhaft, wenn die erfindungsgemäße Zubereitung frei ist von p-Methylbenzylidencampher.

Erfindungsgemäß vorteilhafte Ausführungsformen der vorliegenden Erfindung sind dadurch gekennzeichnet, dass die erfindungsgemäße Zubereitung Glycerin enthält.

Es ist erfindungsgemäß vorteilhaft, wenn die erfindungsgemäße Zubereitung Feuchthaltemittel wie Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure, Hyaluronsäure, Chitosan und/oder Harnstoff enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum.

Es ist erfindungsgemäß bevorzugt, wenn die Zubereitung Xanthangummi und/oder Tapiokastärke enthält.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl wie Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte, Selbstbräuner.

Es ist erfindungsgemäß bevorzugt, wenn die Zubereitung ein oder mehrere Diole gewählt aus der Gruppe der Verbindungen 1,2-Pentandiol, 1,2-Hexandiol, 1,2-Octandiol, 1,2-Decandiol, 2-Methylpropan-1,3-diol enthält.

Die Ölphase der erfindungsgemäßen Zubereitung wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Jojobaöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Phenethylbenzoat, 2-Phenylethylbenzoat, Isopropyl Lauroyl Sarkosinat, Phenyl Trimethicon, Cyclomethicon, Dibutyladipat, Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner vorteilhaft, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Tridecylsalicylat (welches unter der Handelsbezeichnung Cosmacol ESI bei der Fa. Sasol erhältlich ist), C12-C15 Alkylsalicylat (unter der Handelsbezeichnung Dermol NS bei der Fa. Alzo erhältlich), Hexadecylbenzoat und Butyloctylbenzoat und Gemische davon (*Hallstar AB*)*.*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene, C13-16 Isoparaffin und Isohexadecan. Unter den Polyolefinen sind Polydecene die bevorzugten Substanzen.

Die erfindungsgemäßen Zubereitungen können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, dass die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht zyklisch ist und
   - im Bereich von 1 bis 50 mol-% liegt, wenn das Organopolysiloxan zyklisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Vorteilhaft ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Erfindungsgemäß besonders bevorzugte Ausführungsformen der vorliegenden Erfindung sind dadurch gekennzeichnet, dass die Zubereitung eine oder mehrere lipophile Bestandteile gewählt aus der Gruppe der Verbindungen Myristylmyristat, Octyldodecanol, C₁₂-C₁₅-Alkylbenzoat, Butylenglycoldicaprylat/Dicaprat, Ceatylalkohol enthält.

Es ist erfindungsgemäß bevorzugt, wenn die erfindungsgemäße Zubereitung Tocopherylacetat und/oder Tocopherol enthält.

Erfindungsgemäß bevorzugte Ausführungsformen der vorliegenden Erfindung sind dadurch gekennzeichnet, dass die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, ß-Alanin und/oder Licochalcon A, enthält.

Die erfindungsgemäßen Emulsionen können erfindungsgemäß vorteilhaft Methylparaben, Ethylparaben, 2-Methylisothiazol-3(2*H*)-on und/oder Phenoxyethanol enthalten.

Eine alternative Ausführungsform der vorliegenden Erfindung ist jedoch auch dadurch gekennzeichnet, dass die Emusion frei ist von Parabenen.

Es ist erfindungsgemäß vorteilhaft, wenn die erfindungsgemäße Emulsion frei ist von 3-Iodo-2-propynylbutylcarbamat.

Vorteilhaft im Sinne der vorliegenden Erfindung sind Zubereitungen zur Pflege der Haut. Sie können dem kosmetischen Lichtschutz, der Hautpflege (beispielsweise zur Prophylaxe und Behandlung der Hautalterung) und dekorativen Kosmetik dienen.

Die Zubereitungen können sprühbar oder auf einem Träger (z.B. Tuch) dargereicht werden.

Erfindungsgemäß vorteilhaft weist die erfindungsgemäße Zubereitung einen pH-Wert von 5 bis 8 auf. Dieser kann durch die herkömmlichen Säuren, Basen und Puffersysteme eingestellt werden.

### Vergleichsversuch

Der erfinderische Effekt konnte mit dem folgenden Vergleichsversuch belegt werden:

### a) Untersuchung des Dufteindrucks: nach 4 Monaten zeigt sich eine deutliche

Parfümstabilisierung durch die Kombination aus C18-38 Alkyl Hydroxystearoyl Stearate in der Formulierung

| | **Produkt 1** | **Produkt 2** |
|---|---|---|
| **INCI-Name(n)** | **m [%]** | **m [%]** |
| Kaliumcetylphosphat | 0,40 | 0,40 |
| Glycerylstearat | 1,00 | 1,00 |
| Strukturgeber | 1,00 | 1,00 |
| **C18-38 Alkyl Hydroxystearoyl Stearate** | **1,00** | - |
| Ethylhexyl Salicylat | 4,75 | 4,75 |
| Homosalat | 9,50 | 9,50 |
| Weitere UV-Filter wie z.B. Diethylhexyl Butamido Triazone, Titanium Dioxide, Octocrylene, Bis-Ethylhexyloxyphenol Methoxyphenyl Triazine, Phenylbenzimidazole Sulfonic Acid, Butyl Methoxydibenzoylmethan | 18,00 | 18,00 |
| Moisturizer | 0,9 | 0,9 |
| Wirkstoffe | 0,06 | 0,06 |
| Füllstoffe | 1,00 | 1,00 |
| Verdicker | 0,40 | 0,40 |
| Neutralisationsmittel | 0,30 | 0,30 |
| **Parfum enthaltend Coumarin, Limonen, Eugenol und Geraniol** | **0,40** | **0,40** |
| Alcohol Denat. | 6,00 | 6,00 |
| Komplexbildner | 0,20 | 0,20 |
| Konservierungsmittel | 0,90 | 0,90 |
| Wasser | Ad 100,0 | Ad 100,0 |

Parfümprüfungen stellen eine wichtige Voraussetzung für die Einführung neuer Produkte dar, um die Qualität der Produkte zu sichern. Ein Bestandteil eines qualitativ hochwertigen Produktes ist die Stabilität der Parfümierung. Um diese Stabilität zu gewährleisten, werden die entsprechenden Muster über einen Zeitraum von 4 Monaten einlagert.

Die Lagerbedingungen sind: + 6 ° C, Raumtemperatur (RT), Brutschrank 40°C

Mit einer derartigen Messung kann die dreijährige Haltbarkeit von Produkten überprüft werden. Beachtet werden sollten der Füllgrad, die Siegelung, gleiche Verpackungsgröße bzw. Verpackungsart.

Die Muster werden nach 2 und 4 Monaten Lagerung von Parfümevaluatoren olfaktorisch beurteilt. Dazu werden die Muster auf neutrale Pappkarten aufgetragen und im Vergleich bewertet. Das +6°-Muster dient bei der Bewertung der Parfümprüfung als Standard. Entscheidend ist vor allem die Bewertung bei 40°, da hier die Muster besonders gestresst werden.

### Beurteilung von Parfümprüfungen:

| **Note** | **Bewertung in Prozent [%]** | **Zuordnung** |
|---|---|---|
| 1 | 100 | 76 bis 100 % sehr gut |
| 1- | 90 | |
| 2+ | 80 | |
| 2 | 70 | 51 bis 75 % gut / normal |
| 2- | 60 | |
| 3+ | 50 | 26 bis 50 % akzeptabel |
| 3 | 40 | |
| 3- | 30 | |
| 4 | 25 | 0 bis 25 % nicht akzeptabel |

### Ergebnisse:

| **Produkt** | **Lagerungsbedingung** | **nach 2 Monaten** | **nach 4 Monaten** |
|---|---|---|---|
| **1** | 6 °C | 100% | 100% |
| **1** | RT | 100% | 90% |
| **1** | 40 °C | 95% | 85% |
| **2** | 6 °C | 100% | 95% |
| **2** | RT | 90% | 80% |
| **2** | 40 °C | 90% | 75% |

Das Produkt 1 mit C18-38 Alkyl Hydroxystearoyl Stearate und Parfüm enthaltend Coumarin, Limonen, Eugenol und Geraniol zeigt überraschenderweise im Endergebnis nach 4 Monaten Lagerung bei allen Lagertemperaturen +6°C, Raumtemperatur und im Brutschrank bei 40°C eine bessere Dufteindruck/Parfümstabilität als das Produkt 2 ohne Esterwachs.

### b) Verbesserung der Wasserfestigkeit und Erhöhung des Sonnenschutzfaktors

Die Methode zur Bestimmung der Wasserfestigkeit von Sonnenschutzmitteln ist in den im Dezember 2005 von COLIPA veröffentlichten Leitlinien "Guidelines for Evaluating Sun Product Water Resistance" beschrieben.

Der an einem Probanden vor oder nach dem Eintauchen in Wasser bestimmte Lichtschutzfaktor (LSF) eines Sonnenschutzmittels ist definiert als das Verhältnis der minimalen Erythemdosis (MED) auf der geschützten Haut zur MED auf der ungeschützten Haut des gleichen Probanden. LSF und MED werden nach der "Internationalen Methode zur Bestimmung des Lichtschutzfaktors (LSF)" bestimmt. Alle Bestimmungen des LSF vor und nach dem Eintauchen in Wasser müssen im gleichen Labor mit den gleichen Probanden in der gleichen Testsequenz durchgeführt werden.

### Ergebnisse:

| | **Mean Value** | |
|---|---|---|
| | **LSF** | **Water Resistence [%]** |
| **Produkt 1** | **63,8** | **65%** |
| **Produkt 2** | **52** | **39%** |

Die Untersuchung bei einem unabhängigen Institut hat gezeigt, dass beim Einsatz des Esterwachses sowohl der LSF als auch die Wasserfestigkeit deutlich verbessert wurden.

### Beispiel

Das nachfolgende Beispiel soll die vorliegende Erfindung verdeutlichen ohne sie einzuschränken. Die Angaben beziehen sich stets auf Gewichts-%, sofern nicht andere Angaben gemacht werden.

| | |
|---|---|
| | 1 |
| Distarch Phosphate | 1 |
| Kaliumcetylphosphat | 0,3 |
| Glyceryl Stearate | 1 |
| C18-C3B Alkylhydroxystearoylstearat | 1 |
| Myristyl Myristat | 0,5 |
| Butylenglycol Dicaprylat/Dicaprat | 3 |
| Cetearylalkohol | 0,5 |
| Cyclomethicon | 5 |
| Octyldodekanol | 3 |
| Bis-Ethylhexyloxyphenol Methoxyphenyl Triazin | 2,5 |
| Butyl Methoxydibenzoylmethan | 3,5 |
| Homosalat | 9 |
| Octocrylen | 8 |
| Ethylhexylsalicylat | 2 |
| Phenylbenzimidazol Sulfonsäure | 1,5 |
| Tris-(biphenyl)-1,3,5 triazin | 2 |
| Tocopherol Acetate | 0,5 |
| Glycerin | 10 |
| Alcohol Denat. | 4 |
| EDTA | 1 |
| Parfum enthaltend Coumarin, Limonen, Eugenol und Geraniol | 0,8 |
| Phenoxyethanol | 0,6 |
| Neutralisationsmittel (z.B. Sodium Hydroxide) | q.s. |
| Wasser | ad 100 |
| | |
| pH-Wert | 7,5 |

## Patentansprüche

1. Kosmetische Öl-in-Wasser Emulsion (O/W Emulsion) enthaltend
a) C18-C38 Alkylhydroxystearoylstearat,
b) ein oder mehrere UV-Lichtschutzfilter aus der Gruppe der Salicylate Ethylhexylsalicylat und Homomenthylsalicylat (INCI:Homosalate),
c) einen oder mehrere Parfümstoffe,
wobei die Emulsion eine Viskosität von 1000 bis 3000 mPas aufweist gemessen mit Rotationsrheometer Rheomat R123 der Firma proRheo mit dem VT02- Messsystem, wobei die Proben vor der Messung für mindestens 24 Stunden in Ruhe bei 25°C gelagert werden, **dadurch gekennzeichnet, dass** als Emulgator Kaliumcetylphosphat und Glycerylstearat eingesetzt wird.

2. Emulsion nach Anspruch 1, **dadurch gekennzeichnet, dass** die Zubereitung Ethanol enthält.

3. Emulsion nach einem der vorhergehenden Ansprüche, **dadurch gekennzeichnet, dass** der oder die erfindungsgemäßen Parfümstoffe gewählt werden aus der Gruppe Linalool, Benzyl Benzoate, Hydroxyisohexyl 3-Cyclohexene Carboxaldehyd, Hexyl Cinnamal, Benzyl Salicylate, Citronellol, Coumarin, Limonene, Butylphenyl Methylpropional, Eugenol, Geraniol, Alpha-Isomethyl Ionone.

4. Emulsion nach einem der vorhergehenden Ansprüche, **dadurch gekennzeichnet, dass** die Zubereitung weitere UV-Lichtschutzfilter und/oder weitere Parfümstoffe enthält.

5. Emulsion nach einem der vorhergehenden Ansprüche, **dadurch gekennzeichnet, dass** die Zubereitung C18-C38 Alkylhydroxystearoylstearat in einer Gesamtkonzentration von 0,2 bis 2,5 Gewichts-%, bezogen auf das Gesamtgewicht der Zubereitung, enthält.

6. Emulsion nach einem der vorhergehenden Ansprüche, **dadurch gekennzeichnet, dass** die Zubereitung Ethylhexylsalicylat und Homomenthylsalicylat (INCI: Homosalate) in einer Gesamtkonzentration von 3 bis 15 Gewichts-%, bezogen auf das Gesamtgewicht der Zubereitung, enthält, wobei diese Gesamtkonzentration die Einzelkonzentration darstellt für den Fall, dass die Zubereitung nur eines der beiden Salicylate enthält.

7. Emulsion nach einem der vorhergehenden Ansprüche, **dadurch gekennzeichnet, dass** die Zubereitung Ethanol in einer Konzentration von 2 bis 10 Gewichts-%, bezogen auf das Gesamtgewicht der Zubereitung, enthält.

8. Emulsion nach einem der vorhergehenden Ansprüche, **dadurch gekennzeichnet, dass** die Zubereitung Xanthangummi und/oder Tapiokastärke enthält.

9. Emulsion nach einem der vorhergehenden Ansprüche, **dadurch gekennzeichnet, dass** die Zubereitung ein oder mehrere Diole gewählt aus der Gruppe der Verbindungen 1,2-Pentandiol, 1,2-Hexandiol, 1,2-Octandial, 1,2-Decandiol, 2-Methylpropan-1,3-diol enthält.

10. Emulsion nach einem der vorhergehenden Ansprüche, **dadurch gekennzeichnet, dass** die Zubereitung einen oder mehrere weitere UV-Filter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 2-(4'-Diethylamino-2'-hydoxybenzoyl)-benzoesäurehexylester; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)-ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 4-(tert.-Butyl)-4'-methoxydibenzoylmethan; 2-Ethylhexyl-2-hydroxybenzoat;; Dimethicodiethylbenzalmalonat; 3-(4-(2,2-bis Ethoxycarbonylvinyl)-phenoxy)propenyl)-methoxysiloxan / Dimethylsiloxan - Copolymer;) 2-Ethylhexyl-2-cyano-3,3-diphenylacrylat (Octocrylen); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl'-hexy)oxy)]-1,3,5-triazin (INCI: Ethylhexyl Triazone); 2,4-Bis-{[4-(2-ethylhexyloxy)-2-hydroxy]-phenyl}-6-(4-methoxyphenyl)-1,3,5-triazin (INCI: Bis-Ethylhexyloxyphenol Methoxyphenyl Triazin); 2,4,6-Tris-(biphenyl)-1,3,5-triazin; 2,4-Bis-(4'-Di-neopentylaminobenzalmalonat)-6-(4"-butylaminobenzoat)-s-triazin, 4-Dicyanomethylen-2,6-dimethyl-1,4-dihydropyridin-N-(ethyloxysulfatestersalz), Titandioxid, Zinkoxid, Merocyanine, Piperazinderivate, enthält.

11. Emulsion nach einem der vorhergehenden Ansprüche, **dadurch gekennzeichnet, dass** die Zubereitung einen oder mehrere Wirkstoffe gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Carnitin, Carnosin, natürliche und/oder synthetische Isoflavonoide, Glycerylglucose, Kreatin, Kreatinin, Taurin, ß-Alanin und/oder Licochalcon A, enthält.

12. Emulsion nach einem der vorhergehenden Ansprüche, **dadurch gekennzeichnet, dass** die Zubereitung Methylparaben, Ethylparaben, 2-Methylisothiazol-3(2H)-on und/oder Phenoxyethanol enthält.

## Claims

1. Cosmetic oil-in-water emulsion (O/W emulsion) comprising
a) C18-C38 alkyl hydroxystearoyl stearate,
b) one or more UV light protection filters from the group of the salicylates ethylhexyl salicylate and homomenthyl salicylate (INCI: Homosalate),
c) one or more fragrances,
wherein the emulsion has a viscosity of 1000 to 3000 mPas measured with a Rheomat R123 rotary rheometer from proRheo with the VT02 measuring system, wherein the samples are stored undisturbed for at least 24 hours at 25°C before the measurement, **characterized in that** potassium cetyl phosphate and glyceryl stearate are used as emulsifier.

2. Emulsion according to Claim 1, **characterized in that** the preparation comprises ethanol.

3. Emulsion according to either of the preceding claims, **characterized in that** the fragrance(s) according to the invention are selected from the group of linalool, benzyl benzoate, hydroxyisohexyl 3-cyclohexene carboxaldehyde, hexyl cinnamal, benzyl salicylate, citronellol, coumarin, limonene, butylphenyl methylpropional, eugenol, geraniol and alpha-isomethyl ionone.

4. Emulsion according to one of the preceding claims, **characterized in that** the preparation comprises further UV light protection filters and/or further fragrances.

5. Emulsion according to one of the preceding claims, **characterized in that** the preparation comprises C18-C38 alkyl hydroxystearoyl stearate at a total concentration of 0.2 to 2.5 wt% based on the total weight of the preparation.

6. Emulsion according to one of the preceding claims, **characterized in that** the preparation contains ethylhexyl salicylate and homomenthyl salicylate (INCI: Homosalate) at a total concentration of 3 to 15 wt% based on the total weight of the preparation, this total concentration representing the individual concentration in the event that the preparation only comprises one of the two salicylates.

7. Emulsion according to one of the preceding claims, **characterized in that** the preparation comprises ethanol at a concentration of 2 to 10 wt% based on the total weight of the preparation.

8. Emulsion according to one of the preceding claims, **characterized in that** the preparation comprises xanthan gum and/or tapioca starch.

9. Emulsion according to one of the preceding claims, **characterized in that** the preparation comprises one or more diols selected from the group of the compounds 1,2-pentanediol, 1,2-hexanediol, 1,2-octanediol, 1,2-decanediol and 2-methylpropane-1,3-diol.

10. Emulsion according to one of the preceding claims, **characterized in that** the preparation comprises one or more further UV filters selected from the group of the compounds phenylene-1,4-bis(2-benzimidazyl)-3,3',5,5'-tetrasulfonic acid salts; 2-phenylbenzimidazole-5-sulfonic acid salts; 1,4-di(2-oxo-10-sulfo-3-bornylidenemethyl)benzene and salts thereof; 4-(2-oxo-3-bornylidenemethyl)benzenesulfonic acid salts; 2-methyl-5-(2-oxo-3-bornylidenemethyl)sulfonic acid salts; 2,2'-methylenebis(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; hexyl 2-(4'-diethylamino-2'-hydroxybenzoyl)benzoate; terephthalidenedicamphorsulfonic acid; 2-ethylhexyl 4-(dimethylamino)benzoate; amyl 4-(dimethylamino)benzoate; di(2-ethylhexyl) 4-methoxybenzalmalonate; 2-ethylhexyl 4-methoxycinnamate; isoamyl 4-methoxycinnamate; 2-hydroxy-4-methoxybenzophenone, 2-hydroxy-4-methoxy-4'-methylbenzophenone; 2,2'-dihydroxy-4-methoxybenzophenone, 4-(tert-butyl)-4'-methoxydibenzoylmethane; 2-ethylhexyl 2-hydroxybenzoate; dimethicodiethylbenzalmalonate; 3-(4-(2,2-bisethoxycarbonylvinyl)phenoxy)propenyl)methoxy-siloxane / dimethylsiloxane copolymer; 2-ethylhexyl 2-cyano-3,3-diphenylacrylate (octocrylene); dioctylbutylamidotriazone (INCI: Diethylhexyl Butamido Triazone); 2,4-bis[5-(dimethylpropyl)benzoxazol-2-yl-(4-phenyl)imino]-6-(2-ethylhexyl)imino-1,3,5-triazine with CAS No. 288254-16-0; tris(2-ethylhexyl) 4,4',4"-(1,3,5-triazine-2,4,6-triyltriimino)trisbenzoate (also: 2,4,6-tris[anilino(p-carbo-2'-ethyl-1'-hexyloxy)]-1,3,5-triazine (INCI: Ethylhexyl Triazone); 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); 2,4,6-tris(biphenyl)-1,3,5-triazine; 2,4-bis(4'-dineopentylaminobenzalmalonate)-6-(4"-butylaminobenzoate)-s-triazine, 4-dicyanomethylene-2,6-dimethyl-1,4-dihydropyridine-N-(ethyloxysulfate ester salt), titanium dioxide; zinc oxide, merocyanines and piperazine derivatives.

11. Emulsion according to one of the preceding claims, **characterized in that** the preparation comprises one or more active ingredients selected from the group of the compounds glycyrrhetinic acid, urea, arctiin, alpha-lipoic acid, folic acid, phytoene, D-biotin, coenzyme Q10, alpha-glucosylrutin, carnitine, carnosine, natural and/or synthetic isoflavonoids, glycerylglucose, creatine, creatinine, taurine, β-alanine and/or licochalcone A.

12. Emulsion according to one of the preceding claims, **characterized in that** the preparation comprises methylparaben, ethylparaben, 2-methylisothiazol-3-(2H)one and/or phenoxyethanol.

## Revendications

1. Émulsion huile dans eau cosmétique (émulsion H/E), contenant :
a) un stéarate d'alkylhydroxystéaroyle en C18-C38,
b) un ou plusieurs filtres de protection contre la lumière UV du groupe constitué par les salicylates salicylate d'éthylexyle et salicylate d'homomenthyle (INCI : Homosalate),
c) un ou plusieurs parfums,
l'émulsion présentant une viscosité de 1 000 à 3 000 mPas, mesurée avec un rhéomètre rotatif Rheomat R123 de la société proRheo avec le système de mesure VT02, les échantillons étant entreposés au repos à 25 °C pendant au moins 24 heures avant la mesure, **caractérisée en ce que** du cétylphosphate de potassium et du stéarate de glycéryle sont utilisés en tant qu'émulsifiants.

2. Émulsion selon la revendication 1, **caractérisée en ce que** la préparation contient de l'éthanol.

3. Émulsion selon l'une quelconque des revendications précédentes, **caractérisée en ce que** le ou les parfums selon l'invention sont choisis dans le groupe constitué par le linalool, le benzoate de benzyle, l'hydroxyisohexyl-3-cyclohexène-carboxaldéhyde, l'hexylcinnamal, le salicylate de benzyle, le citronellol, la coumarine, le limonène, le butylphényl-méthylpropional, l'eugénol, le géraniol, l'alpha-isométhyl-ionone.

4. Émulsion selon l'une quelconque des revendications précédentes, **caractérisée en ce que** la préparation contient d'autres filtres de protection contre la lumière UV et/ou d'autres parfums.

5. Émulsion selon l'une quelconque des revendications précédentes, **caractérisée en ce que** l'émulsion contient un stéarate d'alkylhydroxystéaroyle en C18-C38 en une concentration totale de 0,2 à 2,5 % en poids, par rapport au poids total de la préparation.

6. Émulsion selon l'une quelconque des revendications précédentes, **caractérisée en ce que** la préparation contient du salicylate d'éthylexyle et du salicylate d'homomenthyle (INCI : Homosalate) en une concentration totale de 3 à 15 % en poids, par rapport au poids total de la préparation, cette concentration totale étant la concentration individuelle lorsque la préparation ne contient qu'un des deux salicylates.

7. Émulsion selon l'une quelconque des revendications précédentes, **caractérisée en ce que** la préparation contient de l'éthanol en une concentration de 2 à 10 % en poids, par rapport au poids total de la préparation.

8. Émulsion selon l'une quelconque des revendications précédentes, **caractérisée en ce que** la préparation contient de la gomme xanthane et/ou de l'amidon de tapioca.

9. Émulsion selon l'une quelconque des revendications précédentes, **caractérisée en ce que** la préparation contient un ou plusieurs diols choisis dans le groupe constitué par les composés 1,2-pentanediol, 1,2-hexanediol, 1,2-octanediol, 1,2-décanediol, 2-méthylpropane-1,3-diol.

10. Émulsion selon l'une quelconque des revendications précédentes, **caractérisée en ce que** la préparation contient un ou plusieurs filtres UV supplémentaires choisis dans le groupe constitué par les composés sels de l'acide phénylène-1,4-bis-(2-benzimidazyl)-3,3'-5,5'-tétrasulfoniqueque ; sels de l'acide 2-phénylbenzimidazole-5-sulfonique ; 1,4-di(2-oxo-10-sulfo-3-bornylidène-méthyl)-benzène et ses sels ; sels de l'acide 4-(2-oxo-3-bornylidène-méthyl)benzène-sulfonique ; sels de l'acide 2-méthyl-5-(2-oxo-3-bornylidène-méthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidène-camphre ; ester hexylique de l'acide 2-(4'-diéthylamino-2'-hydroxybenzoyl)-benzoïque ; acide téréphtalidène-dicamphre-sulfonique; ester 2-éthylhexylique de l'acide 4-(diméthylamino)-benzoïque ; ester amylique de l'acide 4-(diméthylamino)benzoïque ; ester di(2-éthylhexylique) de l'acide 4-méthoxybenzalmalonique ; ester 2-éthylhexylique de l'acide 4-méthoxycinnamique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; 4-(tert.-butyl)-4'-méthoxydibenzoylméthane ; 2-hydroxybenzoate de 2-éthyl-hexyle ; benzalmalonate de diméthicodiéthyle ; copolymère de 3-(4-(2,2-bis-éthoxycarbonylvinyl)-phénoxy)propényl)-méthoxysiloxane/diméthylsiloxane ; acrylate de 2-éthylhexyl-2-cyano-3,3-diphényle (octocrylène) ; dioctylbutylamidotriazone (INCI : Diethylhexyl-Butamidotriazone) ; 2,4-bis-[5-1(diméthylpropyl)benzoxazol-2-yl-(4-phényl)-imino]-6-(2-éthylhexyl)-imino-1,3,5-triazine de n° CAS 288254-16-0) ; ester tris(2-éthylhexylique) de l'acide 4,4',4"-(1,3,5-triazine-2,4,6-triyltriimino)-tris-benzoïque (également : 2,4,6-tris-[anilino-(p-carbo-2'-éthyl-1'-hexyloxy)]-1,3,5-triazine (INCI : Ethylhexyl Triazone) ; 2,4-bis-{[4-(2-éthyl-hexyloxy)-2-hydroxy]-phényl}-6-(4-méthoxyphényl)-1,3,5-triazine (INCI : Bis-Ethylhexyloxyphenol Methoxyphenyl Triazin) ; 2,4,6-tris-(biphényl)-1,3,5-triazine ; 2,4-bis-(4'-di-néopentylaminobenzalmalonate)-6-(4"-butylaminobenzoate)-s-triazine, sel d'ester de N-(éthyloxysulfate) de 4-dicyanométhylène-2,6-diméthyl-1,4-dihydropyridine, dioxyde de titane, oxyde de zinc, mérocyanine, dérivés de pipérazine.

11. Émulsion selon l'une quelconque des revendications précédentes, **caractérisée en ce que** la préparation contient un ou plusieurs agents actifs choisis dans le groupe constitué par les composés acide glycyrrhizique, urée, arctiine, acide alpha-liponique, acide folique, phytoène, D-biotine, coenzyme Q10, alpha-glucosylrutine, carnitine, carnosine, isoflavonoïdes naturels et/ou synthétiques, glycérylglucose, créatine, créatinine, taurine, β-aianine et/ou licochalcone A.

12. Émulsion selon l'une quelconque des revendications précédentes, **caractérisée en ce que** la préparation contient du méthylparabène, de l'éthylparabène, de la 2-méthylisothiazol-3(2H)-one et/ou du phénoxyéthanol.
